# EUROPÄISCHE PATENTSCHRIFT

(11) **EP 0 929 809 B1**
(45) Veröffentlichungstag und Bekanntmachung des Hinweises auf die Patenterteilung: **22.03.2000**
(21) Anmeldenummer: 96943905.8
(22) Anmeldetag: 09.12.1996
(51) Int. Cl.: G01N 33/53, C12N 5/06

(54) **VERFAHREN UND VORRICHTUNG ZUR IN-VITRO DIAGNOSE ALLERGISCHER ERKRANKUNGEN**
PROCESS AND DEVICE FOR IN VITRO DIAGNOSIS OF ALLERGIC DISORDERS
PROCEDE ET DISPOSITIF POUR LE DIAGNOSTIC IN-VITRO DE PATHOLOGIES ALLERGIQUES

(30) Priorität: 08.12.1995 DE 19545920; 11.12.1995 DE 29519602 U
(43) Veröffentlichungstag der Anmeldung: 21.07.1999
(73) Patentinhaber: Raithel, Martin, 92533 Wernberg-Köblitz (DE)
(72) Erfinder: REIMANN, Hans-Jürgen, D-82067 Ebenhausen (DE); RAITHEL, Martin, D-92533 Wernberg-Köblitz (DE)
(74) Vertreter: Kinzebach, Werner, Dr.
(86) Internationale Anmeldenummer: EP9605501
(87) Internationale Veröffentlichungsnummer: WO9722002

(56) Entgegenhaltungen:
- WO-A-90/02741
- SU-A- 1 272 250
- US-A- 4 336 329
- US-A- 4 780 469
- US-A- 5 225 436
- US-A- 5 449 669
- PATENT ABSTRACTS OF JAPAN vol. 017, no. 030 (C-1018), 20.Januar 1993 & JP 04 248981 A (TOSHIBA CORP), 4.September 1992
- PATENT ABSTRACTS OF JAPAN vol. 018, no. 086 (C-1165), 14.Februar 1994 & JP 05 292940 A (SUMITOMO CHEM CO LTD;OTHERS: 01), 9.November 1993

## Beschreibung

Die vorliegende Erfindung betrifft eine tragbare Inkubationsvorrichtung zur Aufrechterhaltung der Vitalität und Funktionalität der Gewebeproben, sowie deren Verwendung zur in-vitro Diagnose allergischer, insbesondere auch pseudoallergischer, immunologischer und umweltbedingter Erkrankungen

Eine vorrangige Aufgabe des menschlichen Immunsystems ist es, körpereigene und körperfremde Substanzen zu unterscheiden und gegebenenfalls die Zerstörung der körperfremden Substanzen einzuleiten.

Die fortschreitende Entwicklung immunologischer Methoden hat in der Vergangenheit immer neue diagnostische und therapeutische Möglichkeiten eröffnet. So nutzt man beispielsweise hochspezifische monoklonale Antikörper als Detektoren für pathogene Viren, Bakterien und Parasiten oder zum Aufspüren pathologischer zellulärer Veränderungen. In jüngster Zeit wurde außerdem festgestellt, daß zahlreiche chronische Erkrankungen auf eine pathologische Reaktion des Immunsystems zurückzuführen sind. Bei den sogenannten Autoimmunkrankheiten wie der Myasthenie, der chronischen Polyarthritis oder auch dem juvenilen Diabetes richtet sich die Immunantwort gegen körpereigene Zellen und Substanzen. Als pathologisch sind auch die Fälle einzuordnen, bei denen der Körper infolge des Kontakts mit einer körperfremden Substanz eine übermäßige Immunantwort auslöst. Diese, als Allergien bezeichneten Überempfindlichkeiten gegenüber an sich harmlosen Stoffen, können Haut- und Schleimhautschwellungen am ganzen Körper oder in schwerwiegenden Fällen lebensbedrohliche Reaktionen in Verbindung mit einem anaphylaktischen Schock auslösen.

Die Aufklärung der individuellen immunologischen Funktionsabläufe, insbesondere die Diagnose der spezifischen Reaktion des einzelnen Patienten auf bestimmte Allergene stellt eine gerade in jüngster Zeit immer wichtiger werdende Aufgabe der Medizin dar.

Für den Nachweis allergischer Reaktionen sind eine Vielzahl unterschiedlichster Verfahren bekannt:

So werden beispielsweise nach Operationen und bei endoskopischen Untersuchungen routinemäßig Gewebeproben entnommen. Nach ihrer Entnahme werden die Gewebeproben nicht mehr mit Nährstoffen versorgt und sterben schnell ab. Mit geeigneten Färbemethoden und immunhistologischen Markierungsverfahren lassen sich aus morphologischen Veränderungen bzw. aus dem Nachweis bestimmter Antikörper Rückschlüsse auf gewisse allergische Reaktionen ziehen. Diese pathohistologischen Verfahren lassen jedoch keine Rückschlüsse auf die Stoffe zu, die eine allergische Sofortreaktion (eine sogenannte Typ-1-Reaktion) auslösen. Ebenso sind keine Aussagen über die aktuelle Funktion der Immunzellen möglich.

Zur Diagnose dieser Typ-1-Allergien werden heute im wesentlichen drei verschiedene Testmethoden eingesetzt:

Am weitesten verbreitet sind die sogenannten Hauttestverfahren. Dabei werden Extrakte verdächtiger Allergene entweder in die Haut eingekratzt, eingestochen oder werden auf die Haut gelegt. Die lokal stimulierte allergische Reaktion äußerst sich dann als Rötung und Anschwellen der betroffenen Hautpartie.

Bei allergischen Reaktionen des Soforttyps ist Immunglobulin E (IgE) als Antikörper beteiligt. Dies macht man sich bei den Bluttestverfahren zunutze, bei denen mit Hilfe von radioimmunologischen oder immunenzymatischen Verfahren die Menge des IgE im Blut bestimmt wird. Diese Verfahren erlauben nicht nur die Bestimmung der Gesamtmenge von IgE im Blut, sondern lassen auch die Bestimmung allergenspezifischer Immunglobuline zu.

Bei der dritten Methode schließlich handelt es sich um sogenannte spezifische Provokationsmaßnahmen, bei denen man die Allergene direkt mit der Schleimhaut des Patienten (beispielsweise der Nasen- oder Darmschleimhaut) in Kontakt bringt und die Reaktion beobachtet.

Ein entscheidender Nachteil aller drei Verfahren ist jedoch die Tatsache, daß der Patient dem Allergen ausgesetzt werden muß. Dies kann einerseits zu einer unerwünschten, erneuten Sensibilisierung führen und andererseits in Einzelfällen sogar schwerwiegende Reaktionen, wie einen anaphylaktischen Schock auslösen.

Es besteht daher ein dringendes Bedürfnis nach einem Test für Typ-1-Allergien (Soforttyp), der einerseits eine zuverlässige Diagnose der Reaktion auf ein spezifisches Allergen erlaubt und der andererseits nicht dazu führt, daß der Patient bei der Untersuchung mit den Allergenen in Kontakt gebracht wird.

In dem US-Patent 5,098,831 ist ein Testverfahren beschreiben, bei welchem Vollblutproben eines Patienten in vitro mit Allergenen stimuliert werden. Der Patient wird dabei nicht mit dem Allergen in Kontakt gebracht. Allergische Reaktionen werden durch den Nachweis von Histamin festgestellt, das durch die im Blut enthaltenen Leukozyten ausgeschüttet wird.

Nachteilig an dem bekannten Verfahren ist jedoch, daß die Histaminbestimmung für sich genommen einen sehr unzuverlässigen Indikator für allergische Reaktionen darstellt. Zum einen lassen sich zahlreiche allergische, pseudoallergische und umweltschadstoffbedingte Erkrankungen durch Untersuchung der Histaminsekretion nicht erfassen bzw. nicht gegeneinander abgrenzen. Zum anderen ist die Bestimmung anderer Indikatoren für allergische Reaktionen in Vollblutproben nicht möglich.

Zur Vermeiung derartiger Nachteile wurde ein Testverfahren vorgeschlagen, das die Stimulation allergischer, pseudoallergischer oder immunologischer Reaktionen in vitalen Gewebeproben in-vitro ermöglicht. Aus Raithel et al., *IntArchAllergyImmunol,* **108**, 127-133 (1995) ist ein Verfahren zur in-vitro Diagnose allergischer Erkrankungen unter Verwendung von vitalen Gewebeproben bekannt, bei dem man das bioptische Gewebe, unmittelbar nachdem man es dem Patienten entnommen hat, in ein temperiertes, sauerstoffhaltiges Inkubationsmedium legt, mindestens ein Allergen zu dem Inkubationsmedium zwecks Stimulation der Gewebeprobe gibt und die Aussschüttung von Immunglobulin E (IgE) oder eines Mediators nach einer bestimmten Einwirkungszeit des Allergens im Inkubationsmedium qualitativ und/oder quantitativ bestimmt. Die nachgewiesenen Konzentrationen von IgE oder eines Mediators werden einer Negativkontrolle, d.h. einer Spontanfreisetzung ohne Allergenstimulation gegenübergestellt.

Bevorzugt handelt es sich bei den bioptischen Gewebeproben um Haut- oder Schleimhautproben, insbesondere um Schleimhautpartikel aus dem Magen-Darm-Trakt des Patienten. Es können allerdings beliebige Haut- oder Schleimhautproben verwendet werden. Diese können insbesondere mittels einer Biopsie beispielsweise der Haut, der Lungen-, der Darm- oder der Magenschleimhaut gewonnen werden.

Überraschenderweise wurde gefunden, daß so komplexe Funktionsabläufe wie allergische oder immunologische Reaktionen auch in-vitro stimuliert werden können. Dabei ergibt sich gegenüber bekannten in-vitro Stimulationsverfahren der Vorteil, daß unterschiedlichste Zellpopulationen an der allergischen Reaktion beteiligt und daher verschiedenste Mediatoren nachweisbar sind.

Das Verfahren besitzt gegenüber der herkömmlich durchgeführten Biopsie den Vorteil, daß die Gewebeprobe nach der Entnahme aus dem Körper des Patienten am Leben gehalten wird, z.B. indem man sie in einem Inkubator mit einem Inkubationsmedium perfundiert. Neben den üblicherweise durchgeführten Tests auf morphologische und pathophysiologische Veränderungen lassen sich auch funktionelle physiologische und immunologische Tests durchführen und es können Schädigungen und Krankheiten ermittelt werden, die sich nicht unmittelbar in morphologischen Veränderungen des Gewebes äußern. Die diagnostischen Möglichkeiten der Biopsie werden so vorteilhaft erweitert. Im Vergleich zu herkömmlichen Allergietests wird deutlich, daß mit diesem Verfahren Allergietests ohne eine Gefährdung des Patienten möglich sind.

Nach Operationen und bei endoskopischen Untersuchungen werden dem Patienten routinemäßig Gewebeproben entnommen. Nach ihrer Entnahme werden die Gewebeproben nicht mehr mit Nährstoffen versorgt und sterben normalerweise schnell ab. Der Pathologe kann aufgrund von makroskopischen und mikroskopischen Veränderungen der Gewebeprobe oder von dünnen Schnitten der Gewebeprobe Rückschlüsse auf bestehende oder auch zurückliegende Erkrankungen des Patienten ziehen.

Mit den üblichen pathohistologischen Methoden lassen sich jedoch keine oder nur sehr unzureichende Aussagen über die Funktionalität der Gewebeproben machen, d.h. insbesondere über die Funktion einzelner Zellen bzw. das Zusammenwirken verschiedener Zellen bei physiologischen Abläufen. Hier sind insbesondere die komplexen Vorgänge bei den oben erwähnten immunologischen oder allergischen Reaktionen zu nennen. So gibt es zwar immunhistologische Markierungsverfahren, bei denen über den Nachweis bestimmter Antikörper in Gewebeschnitten Rückschlüsse auf gewisse allergische Reaktionen gezogen werden können; diese Verfahren versagen aber bei den sog. Typ-1-Reaktionen.

Untersuchungen vitaler Gewebeproben werden bisher in der medizinischen Diagnostik praktisch nicht durchgeführt. Ursache hierfür ist u.a., daß, ähnlich wie in der herkömmlichen Pathohistologie auch, die Untersuchung vitaler Gewebeproben ein aufwendiges Instrumentarium und entsprechend ausgebildetes Fachpersonal benötigt, das üblicherweise nicht an dem Ort vorhanden ist, wo die Gewebeprobe dem Patienten entnommen wird. In der Pathologie stellt dies im allgemeinen kein Problem dar, da sich Organteile oder Gewebeproben beispielsweise in Formalin konservieren lassen und der Pathologe seine Untersuchungen noch Stunden oder sogar Tage später ohne nennenswerten Informationsverlust durchführen kann. Die Proben können also aus Krankenhäusern und von niedergelassenen Ärzten an regionale, spezialisierte Zentren geschickt werden, in denen die eigentlichen Untersuchungen stattfinden.

Eine analoge Vorgehensweise müßte auch bei der Untersuchung von vitalen Gewebeproben in Betracht gezogen werden. Dies scheiterte aber bisher daran, daß bis heute keine geeigneten Vorrichtungen existieren, mit denen Gewebeproben unkompliziert und zuverlässig transportiert werden können, wobei ihre Vitalität in dem Transportbehälter zumindest einige Tage aufrechterhalten bleibt.

Aus US-A-4 336 329 ist eine Inkubationsvorrichtung mit einem äußeren Behälter, einer Tür, einer inneren doppelwandigen Umhüllung, Heizelementen und Anschlüssen für Sauerstoffversorgungsmittel bekannt.

Aus JP-A-04 248 981 und JP-A-05 292 940 sind Inkubationsvorrichtungen bekannt. Sie weisen Mittel zum Einperlen von Luftblasen in einen Probebehälter auf.

Aufgabe der vorliegenden Erfindung ist es daher, eine Inkubationsvorrichtung bereitzustellen, die es ermöglicht, die Vitalität und Funktionalität von Gewebeproben über mehrere Stunden bis hin zu einigen Tagen aufrechtzuerhalten und die so den Transport der Gewebeproben von Krankenhäusern und niedergelasSenen Ärzten zu einzelnen auf entsprechende Untersuchungen spezialisierte Zentren ermöglicht. Die Vorrichtung soll unter Verwendung von vitalen Gewebeproben eine in-vitro Diagnose allergischer und immunologischer Erkrankungen ermöglichen. Dabei soll insbesondere eine Identifikation und gegenseitige Abgrenzung allergischer, pseudoallergischer und umweltschadstoffbedingter Erkrankungen ermöglicht werden. Darüberhinaus soll sich die erfindungsgemäße Vorrichtung für die Untersuchung einer Vielzahl anderer immunologischer Funktionsabläufe eignen. Hierzu gehören insbesondere das Studium entzündlicher Prozeße sowie der Regulationsmechanismen der Immunantwort. Die erfindungsgemäße Vorrichtung soll dabei preisgünstig herstellbar und betreibbar sein, da sich entsprechende Untersuchungsmethoden nur dann durchsetzen werden, wenn die damit verbundenen Kosten so gering wie möglich gehalten werden.

Gegenstand der vorliegenden Erfindung ist somit eine tragbare Inkubationsvorrichtung zur Aufrechterhaltung der Vitalität und Funktionalität von Gewebeproben, mit einem kompakten Gehäuse und einem zugehörigen Deckel, einem in dem Gehäuse angeordneten heizbaren Inkubator für Probenbehälter, die mit einer Nährlösung befüllbar sind, wobei in der Inkubationsvorrichtung außerdem Sauerstoffversorgungsmittel, die über Leitungen ein sauerstoffgashaltiges Fluid in die Nährlösung der Probenbehälter einperlen, und Energieversorgungsmittel, welche die Temperierung des Inkubators gewährleisten, angeordnet sind.

Die einzelnen Probenbehälter nehmen dabei Gewebeproben von einigen Milligramm bis einigen 100 Milligramm Frischgewicht in etwa 2 bis 10 ml Nährlösung auf. Die Temperatur des Inkubators und damit der Nährlösung wird meist bei etwa 37°C konstant gehalten. Der Sauerstoffpartialdruck in der Nährlösung sollte etwa 85 bis 120 mmHg betragen. Aufgrund des geringen Nährlösungsvolumens pro Probenbehälter, ist der Sauerstoffverbrauch äußerst gering, so daß auch die Sauerstoffversorgungsmittel sehr kompakt gestaltet werden können. Die erfindungsgemäße mobile Inkubationsvorrichtung ist daher klein und handlich und kann beispielsweise durch Kurierdienste sicher und zuverlässig von Ärzten und Krankenhäusern zu zentralen Untersuchungslabors transportiert werden.

Als Wärmereservoir des Inkubators kann dabei ein Wasserbad dienen, in das die Probenbehälter eingetaucht werden.

Vorteilhaft weist der Inkubator aber ein Element aus einem festen Material, beispielsweise ein Metallblock auf, in dem Ausnehmungen zur Aufnahme der Probenbehältern ausgespart sind. Damit wird ein sicherer Transport der Inkubationsvorrichtung gewährleistet und Lecks wie sie etwa bei Wasserbädern auftreten können, werden vermieden. Es können jedoch auch Blöcke aus wärmeleitendem Kunststoff zum Einsatz kommen.

In dem Inkubator können Heizelemente, etwa eine Widerstandsheizung, zu dessen Erwärmung vorgesehen sein. Die Heizleistung der Heizelemente wird vorteilhaft über einen Thermostaten gesteuert, so daß die gewünschte Temperatur während der Aufbewahrung des bioptischen Materials möglichst konstant gehalten wird. Die erfindungsgemäße Inkubationsvorrichtung kann auch über Kontrolleuchten verfügen, die anzeigen, ob ein bestimmtes Temperaturintervall während der Aufbewahrung der Proben eingehalten wurde.

Die Sauerstoffversorgungsmittel können eine oder mehrere Patronen eines sauerstoffgashaltigen Fluids umfassen. Dabei kann es sich beispielsweise um Sauerstoff oder einfach um Druckluft handeln. Die Patronen können nachladbar sein und werden in diesem Fall wieder aufgefüllt, bevor die Inkubationsvorrichtung erneut an die Ärzte ausgeliefert wird.

Es ist aber auch möglich anstelle der Sauerstoffpatronen einen Miniaturkompressor oder ein kleines Gebläse vorzusehen, mit dessen Hilfe kontinuierlich Umgebungsluft in die Nährlösungen eingeperlt wird.

Die Sauerstoffversorgungsmittel weisen vorteilhaft eine Leitung auf, die in einer Feinnadeldüse oder einer Fritte in der Nährlösung der Probenbehälter enden. Wesentlich effiziente Sauerstoffversorgung der Nährlösung ist dabei, daß möglichst kleine Gasblasen in die Lösung eingeperlt werden.

Bei einem besonders einfachen Aufbau der erfindungsgemäßen Inkubationsvorrichtung, ist die Feinnadeldüse die Nadel einer Einwegspritze, die ein Teil des Sauerstoffleitungssystems bildet und in den Probenbehälter eingesetzt ist. Der Probenbehälter und das mit der Nährlösung in Kontakt kommende Spritzensystem, können nach jeder Benutzung ersetzt werden, ohne daß dadurch wesentlichen Kosten entstünden.

Als Energieversorgung der gesamten Anordnung kommen bevorzugt Batterien oder aufladbare Akkumulatoren in Frage.

Zur Überwachung der wichtigsten Betriebsparameter verfügt die erfindungsgemäße Inkubationsvorrichtung über wenigstens ein pH-Meter und wenigstens ein Oximeter zur Regelung des pH bzw. des Sauerstoffgehalts der Nährlösung in wenigstens einem der Probenbehälter. Man kann meist davon ausgehen, daß in den übrigen Probenbehältern vergleichbare Bedingungen herrschen. Es ist selbstverständlich aber auch möglich, entsprechende Meßeinrichtungen in allen Probenbehältern vorzusehen. Die Temperatur der Nährlösungen kann direkt in der Lösung oder indirekt über die Temperatur des Inkubators bestimmt werden.

Um den Energieverbrauch der erfindungsgemäßen Vorrichtung so gering wie möglich zu halten bestehen das Gehäuse und der Dekkel aus einem wärmedämmenden Material oder sind zumindest mit einer Isolierschicht verkleidet.

Das vitale bioptische Material ist insbesondere zur in-vitro-Diagnose allergischer Reaktionen verwendbar. Man macht sich dabei entweder zunutze, daß Allergien des Soforttyps mit einer verstärkten Produktion von Immunglobulin E einhergehen, oder daß während des "Allergieanfalls" in der Schleimhaut sogenannte Mediatoren (Botenstoffe) ausgeschüttet werden, welche die eigentlichen Symptome des Allergieanfalls auslösen.

Gegenstand der Erfindung ist daher auch die Verwendung der tragbaren Inkubationsvorrichtung zur in-vitro Diagnose allergischer Erkrankungen nach den beigefügten Ansprüchen 10 - 14.

Häufig bestimmt man auch den Gesamtgehalt an Mediator im Probengewebe. Dazu homogenisiert man die Gewebeprobe zusammen mit einer bestimmten Menge Wasser in einem Homogenisierröhrchen mechanisch, entnimmt festgelegte Aliquote des Homogenats aus dem Homogenisierröhrchen, zentrifugiert diese Aliquote und bestimmt in den resultierenden Überständen den jeweiligen Mediator quantitativ. Gegebenenfalls spült man das Homogenisierröhrchen, um auch die an den Wänden des Röhrchens haftenden Mediatorreste erfassen zu können. Hierzu füllt man das Röhrchen wieder mit der gleichen Wassermenge, homogenisiert wieder mechanisch und entnimmt mengenmäßig die gleichen Aliquote an Lösung aus dem Homogenisierröhrchen. Man zentrifugiert die Aliquote und bestimmt schließlich in den Überständen den jeweiligen Mediator quantitativ. Bevorzugt führt man diesen Spülvorgang des Homogenisierröhrchen mit anschließender Bestimmung der Mediatormenge mindestens zweimal durch, wobei der zweite Spülvorgang mit einer Proteinlösung, insbesondere dem Inkubationsmedium, durchgeführt wird. Aus der Addition der Werte erhält man dann den Gesamtgehalt an Mediator im Probengewebe.

Durch Homogenisierung des stimulierten und nicht stimulierten bioptischen Materials kann neben der Messung der Zellmediatoren auch der Gehalt an Umweltschadstoffen quantifiziert werden. Damit erhält man einen ergänzenden Einblick in die Vitalität der Immunzellen, was Rückschlüssen auf den Immunstatus erlaubt und eine optimierte Therapie gestattet.

Bei den Mediatoren kann es sich um verschiedene, bei allergischen Reaktionen ausgeschüttete Mediatoren handeln, für die an sich bekannte Nachweisverfahren existieren. Bevorzugte untersuchte Mediatoren sind Histamin, Tryptase, ECP (Eosinophil cationic protein), MPO (Myeloperoxidase), DAO (Diaminoxidase), TPS (Tissue polypeptide specific antigen), Interleukine, Prostaglandine oder Zytokine. Der Mediatornachweis kann beispielsweise über ein Radio-Immuno-Assay oder einen ELISA-Test erfolgen. Entsprechende Testverfahren sind kommerziell erhältlich.

Zur Aufrechterhaltung der vollen funktionellen Vitalität der Gewebeprobe wird erfindungsgemäß vorgeschlagen, ein Inkubationsmedium nach Hanks und PIPES/RPMI in modifizierter Form zu verwenden.

Die Hanks(PIPES/RPMI)-Lösung ist kommerziell erhältlich und besitzt im hier bevorzugten Fall einen pH von 6,0 und sollte kein Glycerin, kein Phenolrot, kein MgSO₄ x 7H₂O und kein CaCl₂ x 2H₂O enthalten.

Bei einem bevorzugten Inkubationsmedium enthält die Hanks-Lösung aber zusätzlich 10 bis 40mM Hepes Puffer, 0,5 bis 2% fetales Kälberserum und 0,1-0,5% Humanalbumin. Besonders bevorzugt sind dabei 25mM Hepes Puffer, 1,0% fetales Kälberserum und 0,3% Humanalbumin. Im letzteren Fall entspricht dies beispielsweise 12,5 ml Hepes Puffer, 5 ml fetales Kälberserum und 1,5 g Humanalbumin auf 500ml Hanks-Lösung.

Das neuartige Inkubationsmedium zeichnet sich durch eine hohe Sauerstoffbindungsfähigkeit und eine sehr gute pH-Konstanz während der Begasung mit Sauerstoff aus und besitzt einen hohen Nährwert für das bioptische Material. Das Inkubationsmedium übt keinerlei toxische Wirkung auf das Gewebe aus.

Um möglichst aussagekräftige Resultate zu erhalten, wird das Inkubationsmedium etwa auf Körpertemperatur temperiert, d.h. die Temperatur liegt im Bereich von 37°C. Das Inkubationsmedium wird bevorzugt so mit Sauerstoff begast, daß sich ein Sauerstoff-Partialdruck (pO₂) im Bereich von 85 bis 120 mmHg einstellt. Der Sauerstoff kann aus einer Sauerstofflasche in das Inkubationsmedium eingeperlt werden, bei einer einfacheren Variante wird Umgebungsluft mit Hilfe einer Pumpe, gegebenenfalls über einen Sterilfilter, durch das Inkubationsmedium geleitet. Der pH des Inkubationsmediums wird vorteilhaft im Bereich von 7,4 gewählt.

Mit der erfindungsgemäßen Verwendung sind zahlreiche Vorteile verbunden. Als wichtigster Vorteil ist zu nennen, daß die vollständige immunologische bzw. allergologische Diagnostik in-vitro stattfindet, d.h. der Patient wird bei der Diagnose nicht mehr mit potentiell schädlichen Substanzen belastet. Die einzige Unannehmlichkeit für den Patienten besteht darin, daß eine Schleimhautgewebeprobe entnommen werden muß. Derartige Biopsien werden heutzutage jedoch routinemäßig durchgeführt und sind für den Patienten völlig ungefährlich. Im Gegensatz zu Haut- und Bluttests findet die Stimulation mit dem Allergen in dem Gewebe statt, in dem auch im Körper der allergische Anfall ausgelöst wird. Die Durchführung der Diagnose in-vitro ermöglicht die Einhaltung standartisierter, von Untersuchung zu Untersuchung gleichbleibender Bedingungen und vermeidet so Fehlerquellen, die bei Untersuchungen am menschlichen Körper notwendigerweise auftreten. Gleichzeitig können auch Untersuchungen hinsichtlich der Wirkung bestimmter Medikamente, die man beispielsweise dem Inkubationsmedium zugeben kann, durchgeführt werden.

Ergänzend können genetische Prädispositionen von allergischen Erkrankungen durch molekularbiologische Bestimmung der HLA-Loci nachgewiesen werden, was von großer Bedeutung für die Prophylaxe dieser Erkrankungen ist.

Es lassen sich selbstverständlich entsprechende Untersuchungen auch an tierischen Gewebeproben durchführen. Durch Untersuchung der Zellmediatoren im tierischen Organismus, sowie durch den Nachweis von Umweltschadstoffen im tierischen Gewebe können Rückschlüsse auf eventuelle Auswirkungen auf den menschlichen Organismus durch die Übertragbarkeit dieser Stoffe gezogen werden. Derartige Untersuchungen können beispielsweise dazu beitragen, daß der Mensch, insbesondere die Kinder, schadstofffreier ernährt werden.

Ein typischer Ablauf der in-vitro Diagnose, sowie ein Ausführungsbeispiel der erfindungsgemäßen tragbaren Inkubationsvorrichtung werden im folgenden unter Bezugnahme auf die beigefügten Zeichnungen ausführlicher erläutert.

In der Zeichnung zeigt:
- Figur 1: schematisch die Entnahme einer Gewebeprobe aus einer Schleimhaut eines Patienten;
- Figur 2: die inkubierte Gewebeprobe in einem temperierten Bad;
- Figur 3: das Homogenat aus Gewebeprobe und Inkubationsmedium;
- Figur 4: eine histologische Untersuchung des nicht homogenisierten bioptischen Materials; und schließlich
- Figur 5: einen Querschnitt durch eine erfindungsgemäße tragbare Inkubationsvorrichtung.

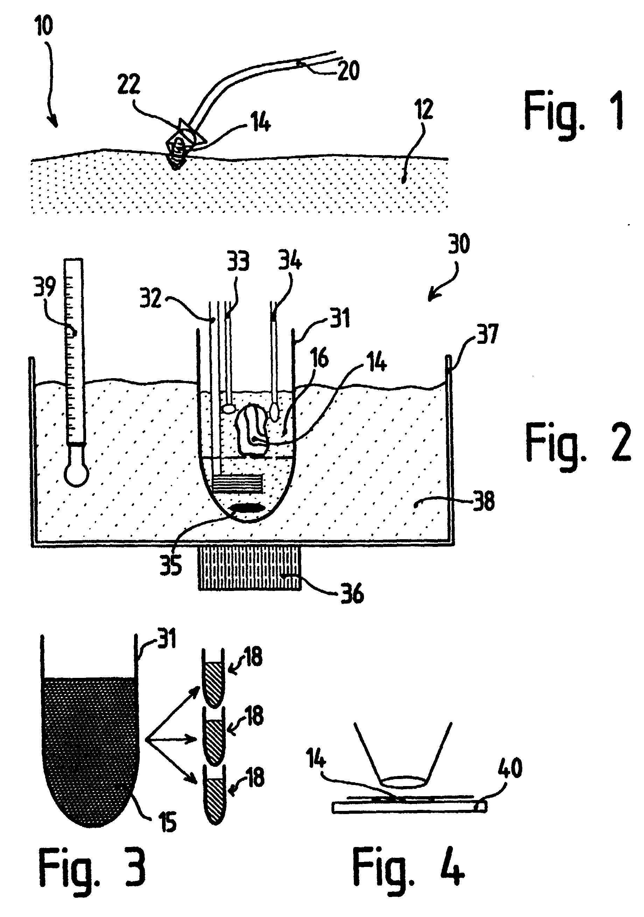

In Fig. 1 ist der Darmabschnitt 10 eines Patienten schematisch dargestellt. Inwandig ist der Darmabschnitt 10 mit einer Schleimhautschicht 12 bedeckt. Ein Endoskop 20 wird in den Darm eingeführt und unter optischer Kontrolle wird über einen Greifarm 22 des Endoskops eine bioptische Gewebeprobe 14 der Darmschleimhaut 12 entnommen. Dabei ist im allgemeinen keine Anästhesierung des Patienten erforderlich.

Eine bioptische Probe hat typischerweise eine Größe von 2 - 5 mm und ein Frischgewicht zwischen 2 und 150 mg.

Wie in Figur 2 dargestellt wird die bioptische Probe 14 unmittelbar nach der Entnahme in einen vorbereiteten Inkubator 30, einen sogenannten Mukosaoxygenator gegeben, wo sie mit einem sauerstoffhaltigen Inkubationsmedium 16 versorgt wird. Als Nährlösung wird ein Hanks- und PIPES/RPMI-modifiziertes Inkubationsmedium verwendet. Etwa 4 ml Inkubationsmedium 16 befinden sich in einem Inkubationsröhrchen 31. Die Sauerstoffversorgung erfolgt über eine Sauerstoffleitung 32, die mit einer am Ende angebrachten Fritte oder Feinnadeldüse in dem Inkubationsmedium endet. Der Sauerstoffgehalt des Inkubationsmediums wird über eine Sauerstoffelektrode 33 bestimmt und bevorzugt im Bereich von 85 bis 120 mmHg über Regulierschrauben eingestellt. Der pH der Lösung wird im Bereich von 7,4 gewählt und über eine in das Inkubationsmedium 16 eintauchende pH-Elektrode 34 kontrolliert. Das Inkubationsröhrchen 31 taucht in das Wasser 38 eines Temperierbades 37 ein. Die Temperatur des Wasserbades ist auf 37°C eingestellt und wird mittels eines Thermometers 39 überwacht. Das Thermometer 39 kann Teil eines Thermostaten sein, wobei zusätzliche (nicht dargestellte) Heiz- bzw. Kühlelemente vorgesehen sein können, die die gewählte Wassertemperatur von 37°C konstant halten.

Die bioptische Probe 14 wird anschließend mit einem Allergen stimuliert. Das Allergen kann in fester, flüssiger oder gasförmiger Form dem Inkubationsmedium 16 zugegeben werden. Es kann aber auch schon vor Einbringen der bioptische Probe 14 in dem Medium 16 vorhanden sein.

Im allgemeinen wird der im Inkubationsmedium aufsteigenden Sauerstoff für eine gute Durchmischung und eine homogene Verteilung des Allergens sorgen. Gegebenenfalls kann jedoch auch ein Miniaturrührfisch 35 am Boden des Röhrchens 31 vorgesehen sein, der von einem außerhalb des Temperierbades angeordneten Magnetrührer angetrieben wird.

Es wird deutlich, daß mit dem Inkubationsverfahren eine allergische Reaktion einer vitalen bioptische Probe 14 hervorgerufen werden kann, ohne daß dabei nachteilige Effekte für den Patienten auftreten können. Ist bereits bekannt, daß ein spezifischer Stoff allergieauslösend wirkt, so kann auch der therapeutische Einfluß bestimmter Medikamente untersucht werden. Beispielsweise kann man dem Inkubationsmedium Antihistaminika oder DNCG (das Dinatriumsalz der Cromoglycinsäure) zugeben, um so den Einfluß bzw. die Wirksamkeit dieser Medikamente bei Stimulation durch das Allergen in-vitro beurteilen zu können.

Nach einer bestimmten Inkubationszeit (üblicherweise nach etwa 240 min) wird die Konzentration des ausgeschütteten Mediators im Inkubationsmedium 16 bestimmt. Ein gewisser Anteil an Mediatorsubstanzen verbleibt jedoch auch im Gewebe 14 selbst. Zur Bestimmung des Gesamtgehalts an Mediator wird die bioptische Probe 14 mechanisch oder mittels Ultraschall homogenisiert. Bei der Homogenisierung kann auch eine enzymatische Behandlung der bioptische Probe erfolgen.

Dazu wird die bioptische Probe aus dem Inkubationsmedium genommen und zunächst auf -72°C abgekühlt, um alle Stoffwechselvorgänge und die einsetzende Abbauprozesse zu unterbinden.

Eine 5 minütige mechanische Homogenisierung einer bioptische Probe mit 2 bis 150 mg Frischgewicht zusammen mit 1500 µl bidestilliertem Wasser bei 1500 Umdrehungen/min liefert beispielsweise 1400 µl Homogenat. Etwa 100 µl gehen erfahrungsgemäß beispielsweise als Spritzer an Wänden und Deckel des Homogenisiergefäßes verloren. Dieser Verlust kann jedoch durch das unten beschriebene Spülen des Homogenisiergefäßes größtenteils wiedergewonnen werden. Das in dem Röhrchen 31 befindliche Homogenat 15 ist in Fig. 3 dargestellt und kann beispielsweise in Aliquote 18 zur weiteren Auswertung aufgeteilt werden. Dabei haben sich, ausgehend von den 1400 µl Homogenat folgende Mengen als vorteilhaft herausgestellt: 100 µl Homogenat unter Zugabe von weiteren 100 µl 8%iger Perchlorsäure für die Histaminbestimmung, 400 µl Homogenat für die Bestimmung von Tryptase, ECP und MPO und schließlich 900 µl für die Bestimmung von DAO, TPS und der Zytokine. Die Aliquote werden anschließend bei 4°C und 7500 Umdrehungen/min für 10 min zentrifugiert. Im Überstand wird die Konzentration der Mediatoren gemessen.

Bei den üblicherweise verwendeten Homogenisiergefäßen handelt es sich um Behälter aus Polytetrafluorethylen (Teflon®), an deren Wänden Mediatorenreste oder ganze Zellen und Zellklumpen des Homogenats haften bleiben können. Durch zweimaliges Spülen ist es möglich, auch diese Reste auszuwerten. Zunächst werden wieder 1500 µl bidestilliertes Wasser in das Homogenisiergefäß gefüllt, aus dem zuvor das Homogenat entnommen wurde. Der mechanische Homogenator wird diesmal für 3 Minuten bei 1500 Umdrehungen pro Minute betätigt. Die aus dem Gefäß entnehmbaren 1400 µl Spüllösung werden in die oben bereits beschrieben Aliquote zur Bestimmung der einzelnen Mediatoren aufgeteilt. Die Aliquote werden bei 4°C und 7500 Umdrehungen/min für 10 min zentrifugiert. Wieder wird im Überstand die Konzentration der Mediatoren gemessen. Der gleiche Spülvorgang des Gefäßes wird noch einmal wiederholt, wobei diesmal 1500 µl einer geeignete Proteinlösung, beispielsweise das Inkubationsmedium selbst, als Spüllösung verwendet werden.

Während das bidestillierte Wasser ein Aufplatzen der Zellen aufgrund des osmotischen Drucks bewirkt, ist das Inkubationsmedium vorallem zum Ablösen der Zellfragmente und Mediatorreste von den Wänden der meist aus Teflon® bestehenden Homogenisiergefäße geeignet.

Falls das spezifische Allergen eine allergische Reaktion ausgelöst hat, erwartet man in dem Inkubationsmedium bzw. im Homogenat eine erhöhte Konzentration von IgE oder von Mediatoren wie Histamin, Interleukine, Prostaglandine oder Zytokine. Zur IgE-Bestimmung werden hauptsächlich die spezifischen Antigene selbst bzw. Anti-IgE-Antikörper eingesetzt. Der bei der Reaktion von IgE mit dem Antigen bzw. dem Anti-IgE-Antikörper entstehende Immunkomplex führt dann wie in vivo zur Allergiereaktion bzw zur Reaktion der Immunzelle.

Alternativ können auch, wie in Fig. 4 dargestellt, konventionelle pathologische bzw. immunhistologische Untersuchungen am Präparat durchgeführt werden.

Routinemäßig werden 5 bis 15 Proben bei einer Endoskopie entnommen und getestet.

Es ist auch möglich den gesamten Test in einem Kontrollexperiment durchzuführen, wobei jedoch keine Allergenstimulation stattfindet. Die dabei erhältlichen Konzentrationen der Mediatoren sind ein Maß für deren Spontanfreisetzung.

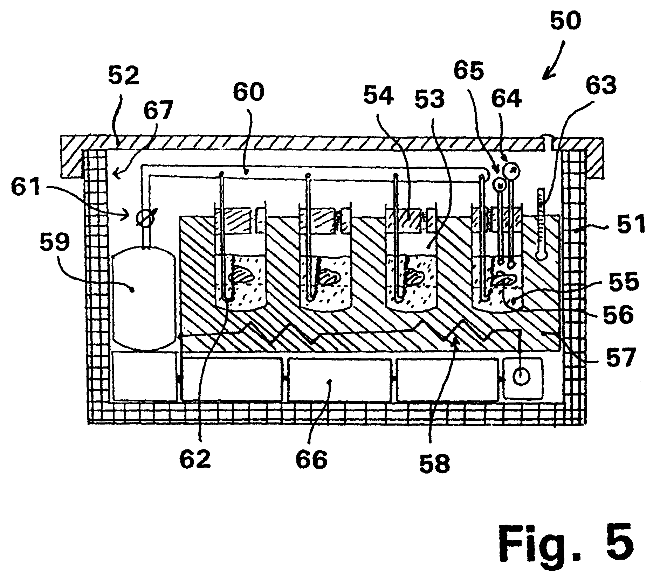

Die Gewebestimulation mit Allergenen kann beispielsweise in der im folgenden beschriebenen und in Fig. 5 dargestellten mobilen Inkubationsvorrichtung durchgeführt werden. Die Inkubationsvorrichtung 50 weist ein kastenförmiges Gehäuse 51 auf, in welchem sich ein als Wärmereservoir dienender Metallblock 57 befindet. In der Oberseite des Metallblocks sind zylindrische Ausnehmungen ausgespart, in denen sich Probenbehälter 53 befinden. Die Probenbehälter sind mit flüssigkeitsdichten Stopfen 54 verschlossen. In den Probenbehältern befindet sich eine vorbereitete Nährlösung 55, die sich insbesondere durch eine hohe Sauerstoffbindungsfähigkeit auszeichnet.

Die Inkubationsvorrichtung 50 enthält weiterhin Heizelemente 58 zum Temperieren des Inkubators 57, eine Sauerstoffversorgung 59, eine Energieversorgung 66, sowie diverse Kontrolleinrichtungen, wie ein oder mehrere Thermometer bzw. einen Thermostat, ein Oximeter und ein pH-Meter.

Der Arzt kann die Inkubationsvorrichtung beispielsweise zusammen mit bereits mit Nährlösung gefüllten Probenbehältern erhalten. Der Inkubator wird eingeschaltet und signalisiert über eine Kontrollampe, wenn die gewünschte Temperatur, beispielsweise 37°C, erreicht ist. Die dem Patienten entnommenen Gewebeproben 56 werden in einzelne Probenbehälter gelegt und an die Sauerstoffversorgung 59 über Leitungen 60 und Feinnadeldüsen 62 angeschlossen. Bei der Sauerstoffversorgung 59 kann es sich um ein kleines Sauerstoffläschchen oder auch eine Sauerstoffpatrone handeln, es ist jedoch auch möglich, ein kleine Gebläse vorzusehen, das Luft, ggf. über einen Sterilfilter, in die Probenbehälter bläst, wobei in diesem Fall der Sauerstoff im wesentlichen der Sauerstoff aus der Luft ist. Über ein Oximeter wird sichergestellt, daß der Sauerstoff-Partialdruck in der Nährlösung im Bereich zwischen etwa 85 und 120 mm Hg liegt. Der pH des Inkubationsmediums liegt etwa im Bereich von 7,4. Ein in der Sauerstoffleitung 60 vorgesehenes Drosselventil 61 gewährleistet die Einhaltung des gewünschten Sauerstoff-Partialdrucks. Die Energieversorgung 66, bevorzugt ein wiederaufladbarer Akkumulator, dient der Stromversorgung der Meß- und Anzeigegeräte sowie der Heizelemente und ggf. des Gebläses. Vorteilhaft ist das Gehäuse 51 mit einem Isoliermantel 67 versehen, und auch der zur Abdeckung des Inkubators verwendete Deckel 52 ist isoliert. Der Energiebedarf der Heizelemente wird dadurch äußerst gering gehalten und der Akku kann entsprechend leicht und platzsparend ausgeführt sein. Der Stopfen 54 des Probenbehälters kann eine semipermeable Membran aufweisen, die den in die Nährlösung eingeperlten Sauerstoff bzw. die Luft durchläßt, die aus der Lösung mitgeführte Feuchtigkeit aber zurückhält. Der Stopfen 54 kann außerdem Durchführungen für die Sauerstoffleitung 60 und gegebenenfalls die Meßleitungen des Oximeters 64 und des pH-Meters 65 aufweisen.

In der Praxis kann beispielsweise ein Kurierdienst eingerichtet werden, der die Inkubatoren mit den Gewebeproben in Krankenhäusern und bei niedergelassenen Ärzten abholt und dort einsatzbereite Inkubationsvorrichtungen mit frische Nährlösung enthaltenden Probenbehältern, aufgeladenen Batterien und aufgeladener Sauerstoff- oder Luftpatrone wieder abgibt. Es versteht sich, daß die erfindungsgemäßen tragbaren Inkubationsvorrichtungen verschiedenste Größen aufweisen können, d.h. insbesondere eine unterschiedliche Anzahl von Probenbehältern enthalten können.

## Patentansprüche

1. Tragbare Inkubationsvorrichtung zur Aufrechterhaltung der Vitalität und Funktionalität von Gewebeproben, mit einem kompakten Gehäuse (51) und einem zugehörigen Deckel (52), einem in dem Gehäuse angeordneten heizbaren Inkubator (57) für Probenbehälter (53), die mit einer Nährlösung befüllbar sind, wobei in der Inkubationsvorrichtung außerdem Sauerstoffversorgungsmittel (59), die über Leitungen (60) ein sauerstoffgashaltiges Fluid in die Nährlösung der Probenbehälter (53) einperlen, und Energieversorgungsmittel (66), welche die Temperierung des Inkubators (57) gewährleisten, angeordnet sind.

2. Inkubationsvorrichtung gemäß Anspruch 1, dadurch gekennzeichnet, daß der Inkubator (57) als Wärmereservoir ein Wasserbad oder einen Metallblock mit Ausnehmungen zur Aufnahme der Probenbehälter (53) aufweist.

3. Inkubationsvorrichtung gemäß einem der Ansprüche 1 oder 2, dadurch gekennzeichnet, daß in dem Inkubator (57) Heizelemente (58) zu dessen Erwärmung vorgesehen sind, deren Heizleistung über einen Thermostaten (63) gesteuert wird.

4. Inkubationsvorrichtung gemäß einem der Ansprüche 1 bis 3, dadurch gekennzeichnet, daß die Sauerstoffversorgungsmittel (59) eine oder mehrerer Patronen eines sauerstoffgashaltigen Fluids umfassen.

5. Inkubationsvorrichtung gemäß einem der Ansprüche 1 bis 3, dadurch gekennzeichnet, daß die Sauerstoffversorgungsmittel (59) einen Miniaturkompressor oder ein kleines Gebläse umfassen.

6. Inkubationsvorrichtung gemäß einem der Ansprüche 1 bis 5, dadurch gekennzeichnet, daß die Sauerstoffversorgungsmittel (59) eine Leitung (60) aufweisen, die in einer Feinnadeldüse (62) oder einer Fritte in der Nährlösung (55) des Probenbehälters (53) endet.

7. Inkubationsvorrichtung gemäß Anspruch 6, dadurch gekennzeichnet, daß die Feinnadeldüse (62) die Nadel einer Einwegspritze ist, die einen Teil des Sauerstoffleitungssystems (60) bildet.

8. Inkubationsvorrichtung gemäß einem der Ansprüche 1 bis 7, dadurch gekennzeichnet, daß die Energieversorgungsmittel (66) Batterien oder aufladbare Akkumulatoren umfassen.

9. Inkubationsvorrichtung gemäß einem der Ansprüche 1 bis 8, dadurch gekennzeichnet, daß sie wenigstens ein pH-Meter und wenigstens ein Oximeter zur Überwachung des pH bzw. des Sauerstoffgehalts der Nährlösung in wenigstens einem der Probenbehälter (53) aufweist.

10. Verwendung einer mobilen Inkubationsvorrichtung nach einem der Ansprüche 1 bis 9 zur in-vitro Diagnose allergischer Erkrankungen durch Allergenstimulation vitaler bioptischer Gewebeproben.

11. Verwendung gemäß Anspruch 10, wobei man
- das bioptische Gewebe, unmittelbar nachdem man es dem Patienten entnommen hat, in ein in einem Probebehälter der Inkubationsvorrichtung befindliches temperiertes, sauerstoffhaltiges Inkubationsmedium legt;
- mindestens ein Allergen zu dem Inkubationsmedium zwecks Stimulation des bioptischen Gewebes gibt; und
- die Aussschüttung von Immunglobulin E (IgE) oder eines Mediators nach einer bestimmten Einwirkungszeit des Allergens im Inkubationsmedium qualitativ und/oder quantitativ bestimmt.

12. Verwendung nach Anspruch 11, wobei die Mediatoren ausgewählt sind unter Histamin, Tryptase, ECP, MPO, DAO, TPS, Interleukine, Prostaglandine oder Zytokine.

13. Verwendung nach einem der Ansprüche 11 oder 12, wobei das Inkubationsmedium ein Inkubationsmedium nach Hanks und PIPES/RPMI ist, modifiziert durch einen Zusatz bestehend aus:
| | |
|---|---|
| 10-40mM | Hepes Puffer, |
| 0,5-2% | fetales Kälberserum und |
| 0,1-0,5% | Humanalbumin. |

14. Verwendung nach Anspruch 13, wobei der Hanks-Lösungszusatz aus
| | |
|---|---|
| 25mM | Hepes Puffer, |
| 1,0% | fetales Kälberserum und |
| 0,3% | Humanalbumin |
besteht.

## Claims

1. A portable incubation device for maintaining the vitality and functioning of tissue samples, comprising a compact housing (51) and a lid (52) associated thereto, a heatable incubator (57), arranged in said housing, for sample containers (53) which can be charged with a nutrient solution, wherein said incubation device further comprises oxygen-supply means (59) which bubble a fluid containing oxygen gas through lines (60) into said nutrient solution in the sample containers (53), and energy-supply means (66) which ensure control of the incubator (57) temperature.

2. An incubation device as claimed in Claim 1, wherein said incubator (57) has, as heat reservoir, a water bath or a metal block with recesses to receive said sample containers (53).

3. An incubation device as claimed in either of Claims 1 or 2, wherein heating elements (58) are provided in said incubator (57) for heating thereof, their heat output being controlled by a thermostat (63).

4. An incubation device as claimed in any of Claims 1 to 3, wherein said oxygen-supply means (59) comprise one or more cartridges of a fluid containing oxygen gas.

5. An incubation device as claimed in any of Claims 1 to 3, wherein said oxygen-supply means (59) comprise a miniature compressor or a small blower.

6. An incubation device as claimed in any of Claims 1 to 5, wherein said oxygen-supply means (59) have a line (60) which terminates in a fine-needle nozzle (62) or a sintered disk in the nutrient solution (55) in the sample container (53).

7. An incubation device as claimed in Claim 6, wherein said fine-needle nozzle (62) is the needle of a disposable syringe which forms part of said oxygen line system (60).

8. An incubation device as claimed in any of Claims 1 to 7, wherein said energy-supply means (66) comprise batteries or chargeable accumulators.

9. An incubation device as claimed in any of Claims 1 to 8, which comprises at least one pH-meter and at least one oximeter to monitor respectively the pH and the oxygen content of said nutrient solution in at least one of said sample containers (53).

10. The use of a mobile incubation device as claimed in any of Claims 1 to 9 for the in vitro diagnosis of allergic disorders by allergen stimulation of vital biopsy tissue samples.

11. The use as claimed in Claim 10, which comprises
- placing the biopsy tissue immediately after it has been taken from the patient in a temperature-controlled, oxygen-containing incubation medium located in a sample container of the incubation device,
- adding at least one allergen to the incubation medium for the purpose of stimulating the biopsy tissue, and
- determining qualitatively and/or quantitatively the immunoglobulin E (IgE) or a mediator released into the incubation medium after the allergen has acted for a certain time.

12. The use as claimed in Claim 11, wherein the mediators are selected from histamine, tryptase, ECP, MPO, DAO, TPS, interleukins, prostaglandins or cytokines.

13. The use as claimed in either of Claims 11 or 12, wherein the incubation medium is a Hanks and PIPES/RPMI incubation medium modified by an addition consisting of
10-40 mM Hepes buffer,
0.5-2% fetal calf serum and
0.1-0.5% human albumin.

14. The use as claimed in Claim 13, wherein the Hanks solution addition consists of
25 mM Hepes buffer,
1.0% fetal calf serum, and
0.3% human albumin.

## Revendications

1. Dispositif d'incubation portable pour maintenir la vitalité et la fonctionnalité de prélèvements tissulaires, avec un boîtier compact (51) et un couvercle (52) lui appartenant, un incubateur (57) pouvant être chauffé, disposé dans le boîtier, pour des récipients (53) de prélèvement, qui peuvent être remplis d'une solution nutritive, dispositif d'incubation dans lequel sont disposés de plus des moyens d'alimentation (59) en oxygène qui introduisent un fluide contenant de l'oxygène gazeux, en le faisant barboter, dans la solution nutritive des récipients de prélèvement (53) par l'intermédiaire de conduites (60), et des moyens d'alimentation en énergie (66), qui assurent l'équilibrage de la température de l'incubateur (57).

2. Dispositif d'incubation selon la revendication 1, caractérisé en ce que l'incubateur (57) comprend comme réservoir de chaleur, un bain-marie ou un bloc métallique avec des évidements pour recevoir les récipients de prélèvement (53).

3. Dispositif d'incubation selon l'une des revendications 1 ou 2, caractérisé en ce que des éléments chauffants (58) sont prévus dans l'incubateur (57) pour le chauffer et dont la puissance de chauffage est commandée par un thermostat (63).

4. Dispositif d'incubation selon l'une quelconque des revendications 1 à 3, caractérisé en ce que les moyens d'alimentation en oxygène (59) comportent une ou plusieurs cartouches de fluide contenant de l'oxygène gazeux.

5. Dispositif d'incubation selon l'une quelconque des revendications 1 à 3, caractérisé en ce que les moyens d'alimentation en oxygène (59) comportent un compresseur miniature ou une petite soufflerie.

6. Dispositif d'incubation selon l'une quelconque des revendications 1 à 5, caractérisé en ce que les moyens d'alimentation en oxygène (59) comprennent une conduite (60) qui se termine par une buse à aiguille fine (62) ou par une fritte dans la solution nutritive (55) du récipient de prélèvement (53).

7. Dispositif d'incubation selon la revendication 6, caractérisé en ce que la buse à aiguille fine (62) est l'aiguille d'une seringue à usage unique, qui forme une partie du système d'alimentation en oxygène (60).

8. Dispositif d'incubation selon l'une quelconque des revendications 1 à 7, caractérisé en ce que les moyens d'alimentation en énergie (66) comportent des piles ou des accumulateurs rechargeables.

9. Dispositif d'incubation selon l'une quelconque des revendications 1 à 8, caractérisé en ce qu'il comprend au moins un pH-mètre et au moins un oxymètre pour contrôler le pH ou la teneur en oxygène de la solution nutritive dans au moins un des récipients de prélèvement (53).

10. Utilisation d'un dispositif d'incubation mobile selon l'une quelconque des revendications 1 à 9, pour le diagnostic in vitro de maladies allergiques à l'aide d'une stimulation allergénique de prélèvements tissulaires biopsiques vitaux.

11. Utilisation selon la revendication 10, au cours de laquelle
- on place le tissu biopsique, directement après son prélèvement chez le patient, dans un milieu d'incubation, contenant de l'oxygène, équilibré en température, se trouvant dans un récipient de prélèvement du dispositif d'incubation ;
- on ajoute au moins un allergène au milieu d'incubation afin de stimuler le tissu biopsique ; et
- on détermine quantitativement et/ou qualitativement la distribution de l'immunoglobobuline E (IgE) ou d'un médiateur après une certaine durée de l'effet de l'allergène.

12. Utilisation selon la revendication 11, dans laquelle les médiateurs sont choisis parmi l'histamine, la tryptase, ECP, MPO, DAO, TPS, les interleukines, les prostaglandines ou les cytokines.

13. Utilisation selon la revendication 11 ou 12, dans laquelle le milieu d'incubation est un milieu d'incubation selon Hanks et PIPES/RPMI, modifié par un appoint constitué de :
10 à 40 mM de tampon HEPES,
0,5 à 2% de sérum bovin foetal et
0,1 à 0,5% d'albumine humaine.

14. Utilisation selon la revendication 13, dans laquelle l'appoint à la solution de Hanks est constitué de :
25 mM de tampon HEPES,
1% de sérum bovin foetal et
0,3% d'albumine humaine.
